# EUROPEAN PATENT SPECIFICATION

(11) **EP 1 700 561 B1**
(45) Date of publication and mention of the grant of the patent: **16.01.2013**
(21) Application number: 03759057.7
(22) Date of filing: 27.10.2003
(51) Int. Cl.: A61B 1/303, G01N 21/62, A61B 5/00

(54) **ACTINIC LIGHT COLPOSCOPE FOR SPECIFIC DETECTION OF LESIONS CAUSED BY THE HUMAN PAPILLOMA VIRUS IN THE LOWER FEMALE GENITAL TRACT**
AKTINISCHES LICHT-KOLPOSKOP FÜR DEN SPEZIFISCHEN NACHWEIS VON LÄSIONEN, DIE DURCH DAS MENSCHLICHE PAPILLOMA-VIRUS VERURSACHT WERDEN, IM UNTEREN GENITALTRAKT DER FRAU
DETECTION SPECIFIQUE DE LESIONS PRODUITES PAR LE VIRUS DU PAPILLOME HUMAIN DANS LE TRACTUS GENITAL FEMININ INFERIEUR

(43) Date of publication of application: 13.09.2006
(73) Proprietor: AVM Licensing Corporation, Merritt Island, FL 32953 (US)
(72) Inventor: Diaz Sanchez, Joel Gerardo, Delegacion Cuauhtemoc, 06140 México (MX); Zertuche Zuani, Joes, Gerardo, Delegacion Cuauhtemoc, Mexico, D.F. 06700 (MX)
(74) Representative: Gee, Steven William
(86) International application number: PCT/MX2003/000088
(87) International publication number: WO 2005/039403

(56) References cited:
- EP-A2- 1 067 376
- EP-A2- 1 161 919
- WO-A2-01/72216
- US-B1- 6 277 067
- DATABASE WPI Week 200028, Derwent Publications Ltd., London, GB; Class P31, AN 2000-322203, XP002999770 & JP 2000 097859 A (OLYMPUS OPTICAL)
- DATABASE WPI Week 200332, Derwent Publications Ltd., London, GB; Class S03, AN 2003-339690, XP002999771 & RU 2 187 116 C2
- DATABASE WPI Week 199230, Derwent Publications Ltd., London, GB; Class B04, AN 1992-062689, XP002999772 & SU 1 393 085 A1
- DATABASE WPI Week 198303, Derwent Publications Ltd., London, GB; Class E23, AN 1983-06556K, XP002999773 & SU 911 254 A1

## Description

### Technical Field of the Invention.

Gynecological tools, gynecological detection tools to detect lower female genital tract lesions, actinic light-based tools to detect lower female genital tract lesions.

### BACKGROUND OF THE INVENTION

The viral gender Papilloma is an extremely extensive DNA virus which infects many animal species populations, including humans. There are over 100 types of known Papilloma viral sources, which when infecting humans are known as Human Papilloma Virus (HPV). The interest in those viruses has been gradually increasing since 1970, when for the first time a function was attributed in the etiology of the Uterine Cervical Cancer.

Cervical Uterine Cancer is a common type of cancer in women which results from a change of the epithelial cell tissue of the cervix, vaginal walls and vulva. Those cells are normal initially, and gradually turn pre-cancerous. Before cancerous cells are found in the cervix, the cervical tissue undergoes changes and abnormal cells start to appear. The process is known as Dysplasia or Cervical Intraepithelial Neoplasia ("CIN").

More than 30 types of viral HPV exist which can infect the female low genital tract. There are benign types of these viruses that are called "low risk," and other oncogenics called "high risk."

Uterine Cervical Cancer is one of the most common forms of cancers among women. It is estimated that there are over 500,000 cases of Uterine Cervical Cancer per year worldwide. Nearly 80% of these cases are reported in developing countries. The high incidence of Uterine Cervical Cancer reflects the deficiencies of early detection programs.

The high risk type of virus in the American Continent has been the HPV 16; identified as Asian-American variety in about 50% of the reported cases.

Starting in 1990 there has been an increase in the research geared toward early detection of Uterine Cervical Cancer. New detection methods are being tested such as fluorescence spectroscopy, cytological techniques, and various testing methods using molecular biology.

The tool used for the observation and diagnosis of many female lower genital tract diseases is called colposcope. The examination/detection technique using a colposcope is known as colposcopy. More specifically, colposcopy is the procedure used in gynecology to examine the epithelium of the female inferior genital tract using the accumulated knowledge in the field to evaluate its illnesses. A colposcope is similar to a stereoscopic microscope, to the extent that it is an optical system that modulates the photogenic energy through which it enlarges an image. The colposcope was invented in 1925 in Germany.

The colposcopy technique generally comprises the steps of: (1) inserting a vaginal mirror whilst the patient is in the gynecological position; (2) placing the colposcope a few centimeters off the vaginal introitus in such a way that the tool does not touch the patient; and (3) observing the desired tissue, its morphology and its vascular patterns.

The prior art documents WO-A-01-72216 and JP-2000-097 859 each disclose a colposcopy apparatus adapted to detect cervical cancer, and share the features of the preamble of claim 1.

One of the biggest problems presented by the present state of the art is that HPV induced lesions and CINs are not often easily diagnosed using the procedure outlined above. The main reason is that the common clinical environment usually lacks the equipment and therefore the tests to facilitate the easy diagnosis of those conditions. Accordingly, many gynecologists have to use laboratory tests with wide margins of error, or use highly sophisticated and costly equipment, e.g., spectroscopy. Such equipment usually requires a large investment of time and resources to train personnel, long waiting periods to obtain results and multiple visits by the patient.

The present invention presents a radical advance in the field, as well as a practical solution to the shortcomings of the prior art in the field of instrumentation to diagnose of lesions caused by HPV. As set forth above, the HPV is the etiological agent of most neoplasias and uterine cervical cancers. Uterine cervical cancer is the number one cause of death through cancer among women between the third and sixth decade of life. That disease is also the second most frequent malignant neoplasia in the world, with an incidence in the industrialized countries of 10 per 100,000 inhabitants. In developing countries the incidence quadruples to 40 per 100,000 inhabitants. More specifically, in Mexico among women older than 25 years of age, the incidence of uterine cervical cancer is 50 per 100,000 inhabitants and 16,000 new cases are detected each year according to the statistics of the Mexican Health Department. Every 2 hours a Mexican woman dies of Cervical Cancer. In short, the HPV is the most important risk factor for CIN.

The apparatus of the present invention avoids costly medical tests and provides quick and highly reliable diagnostic results for the detection of HPV in the female lower genital tract. In fact, the present invention allows excellent and reliable results to be obtained at the time the tests are being conducted and in the same location.

There are additional, districts advantages presented by the apparatus of the present invention, among them: (1) portability; (2) ergonomic design for the user and patient; (3) ease of use; (4) low heat intensity light source; (5) works dually like a common colposcope and like an actinic line colposcope; and (6) is specific to detecting lesions caused by HPV by virtue of its spectral band with a long-lasting light with a specific wave for the fluorochrome fluorescein isothiocyanate ("FITC"); (7) quick results during the same visit and at the same location where the test is performed; and (8) significantly lower costs and less taxing testing requirements for user and patient alike.

### SUMMARY OF THE INVENTION

The description herein below, as well and the accompanying figures, clearly describe in detail the characteristics of the new and useful apparatus of the present invention.

The apparatus of the present invention is set out in claim 1 and comprises mechanical, electrical and optical (also referred to herein as the actinic light) components.

The mechanical component can further comprise a base or support, an arm, and a bolster. The base or support can comprise a rectangular steel plate, which provides overall stability to the equipment; four independent rotating wheels further comprising a brake mechanism which allows the equipment to be moved and set in place; a horizontal support further comprising a rotating articulation to maneuver and set the optical handle and bolster, and further holding in place the feeding source for the illumination equipment.

The arm can comprise a counterbalanced mechanism which allows tri-axial movement, which in turn allows the user of the present invention to easily set the equipment in the precise location required by the gynecological procedure being conducted.

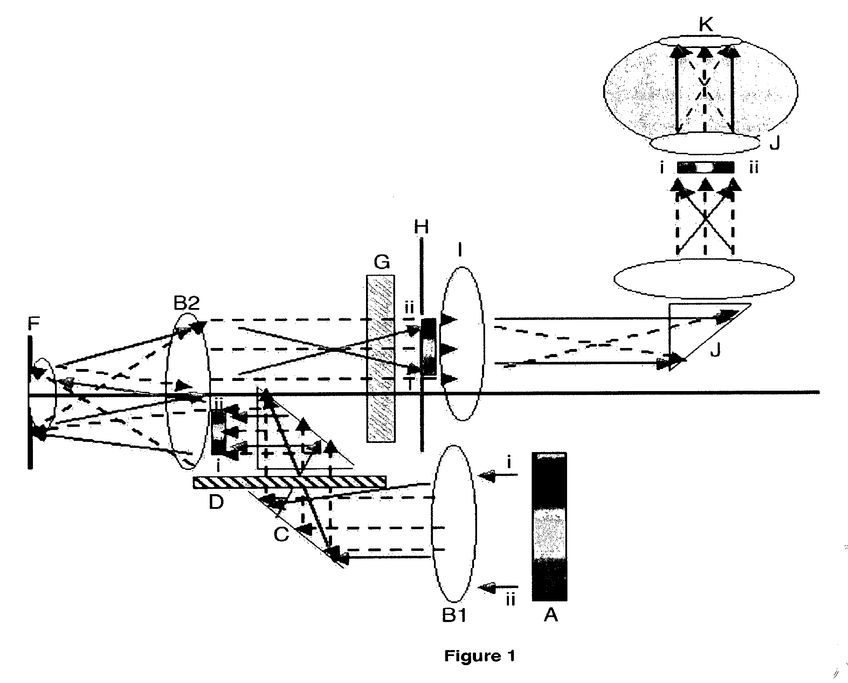

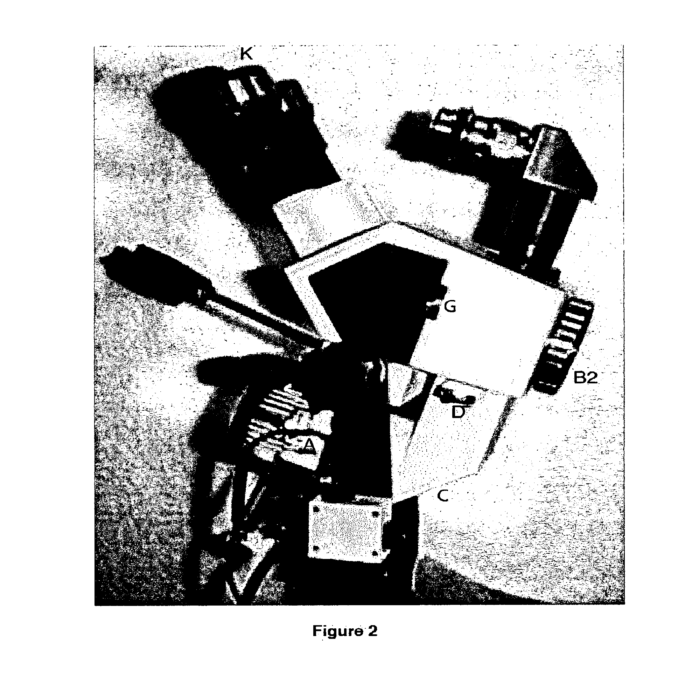

The bolster can be the central and, in some respects, the most functionally important element of the apparatus, as illustrated in Figures 1 and 2. The bolster can contain most of the elements that constitute the "new" colposcope. The bolster can generally comprise: (1) a focusing system which further comprises a mechanism which allows finely calibrated movement; and (2) an optical source of actinic light which further comprises an exciting filter (Figures 1-D and 2-D), and suppressing filters (Figures 1-G and 2-G). The bolster can be a fundamental component of the apparatus of this invention because therein takes place the transmission and modulation of luminescent energy, which allows the user to see cervical images which can then be photographed.

The electronic component of the present invention can comprise: (1) a power source that feeds a bulb (halogen photo optic lamp 12 volts 50 watts); (2) two connectors of 120 V, alternating current suitable for a video camera and recorder; (3) and a contact of 12 V, direct current for a frontal lamp; and (4) sufficient energy controlling means which allow regulation of the required energy level and reading via a voltmeter The electronic component can work with both direct and alternate current sufficiently meeting the energy requirements of the apparatus of this invention. All the elements in the electronic component are contained inside a control box.

The optical and actinic light component can comprise (1) a halogen light bulb (Figure 1-A and 2-A); (2) a collector lens with the halogen light bulb being positioned vertically and parallel to the collector lens (Figure 1- B1); (3) a means to position the light bulb which allows adjustment of the distance in two axes until the highest luminescent intensity is obtained according with Kohler's luminescence characteristics described herein below.

The method of use of the present invention comprises the general steps of: (1) illumination, (2) excitement, and (3) suppression.

During the illumination step, the light emitted by the light bulb's filament (Figure 1-A) can pass through the collector lens (Figure 1-B1) which focuses the light beam which, in turn, is reflected at 90 degrees through a mirror placed for that purpose at 45 degrees in the axis of the light's trajectory (Figure 1-C and 2-C). It cannot be overemphasized that the apparatus of the present invention results in an episcopic illumination. Episcopic illumination is based on the illumination norms which adhere to optical and microscopic principles, including Kohler's illumination. Kohler's illumination allows the use of luminescent energy with minimal waste and heat emission.

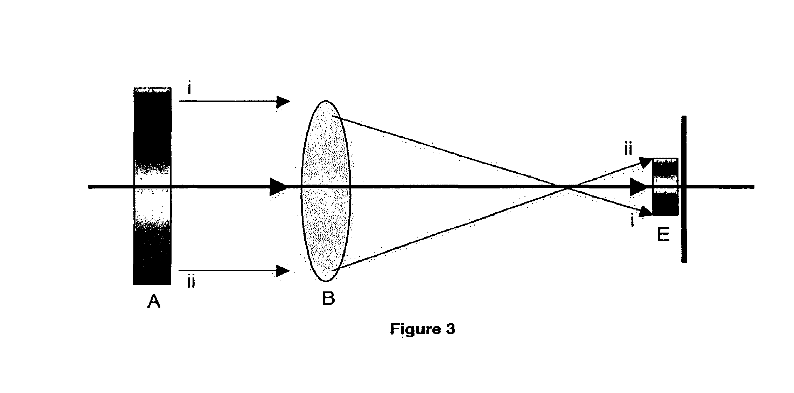

The light source of this invention can be a luminescent source focused to infinity (Figure 1-A and 3-A). It can pass through a convergent or positive lens (Figure 1-B1 and 3-B), which deviates the light beams and concentrates them in a focal plane (Figure 3-E) resulting in an inverted image (Figure 3-i and 3-ii) of a smaller diameter.

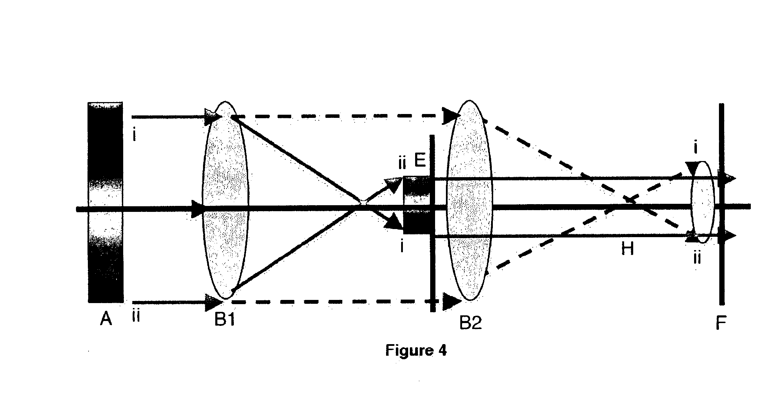

When two convergent collector lenses are used (Figure 4), the second collector lens in front of the objective (Figure 1-B2, 2-B2 and 4-B2), concentrates the image projected by the first collector lens (Figure 1-B1 and 4-B1), the image of the filament of the light source goes through without being seen or perceived at the focal concentrating plane, which will be the cervix (Figure 4-F). In order to understand the present invention one must take into account that the bulb's image comprises a spiral filament which passes through without being seen. The filament is also invisible, but its energy can be used in an optimal manner because its beams in the second focal plane cross in a parallel manner (Figure 4-H) contrary to the projected image of the first convergent collector lens (Figure 4-1 and 4-ii). The above described steps comprise Kohler's principle.

The first lens' image is projected on the focal plane's surface (uterine cervix). Smooth and colorless like crystal (Figures 1-F, 4-F), the image looks like a uniform light field.

To fully understand the excitement state, one must first discern what "light" is. Our surroundings are full of waves. Humans can see or hear through waves, but our limited sensory capacity prevents us from detecting a vast array of waves. In the submicroscopic world, atoms and molecules are constituted of electrons, protons, mesotrons, photons, etc., which move in such a manner as to form waves in their bonds. Appropriately excited, those atoms and molecules emit waves that we call gamma rays, x-rays, ultraviolet waves, visible waves, television, radio, radiation and infrared, etc. (Electromagnetic Spectrum). The movements that are repeated by themselves on regular intervals are called periodic movements. Periodic movements include the sound of a pendulum clock, or the sound of a diapason. The vibration frequency is defined as the number of vibrations in one second. In other words, the frequency of vibration (v) and the period (T) they are reciprocals of each another: Frequency = 1/Period and Period = 1/Frequency. The same relationship expressed in algebraic symbols would be: v = 1/T and T = 1/v.

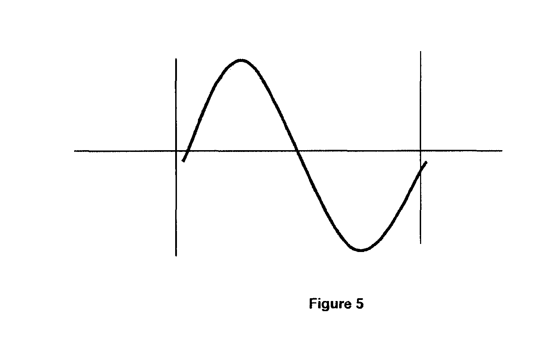

The movement of a body in graphic terms is often expressed in revolutions per minute (rpm), or revolutions per second (rps). Accordingly:
1 cycle / second = 1 vibration / second = 1 Hertz (Hz)
1 Hertz (Hz) is the unit.
Waves are described by their amplitude (crest to crest), frequency (Hz) and wavelength denominated λ (Figure 5).

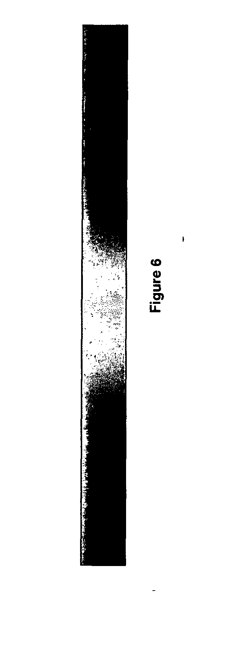

In the electromagnetic spectrum, light waves are designated according to their wavelength, which is measured in nanometers (nm). One nanometer equals one billionth of a meter, or 10⁻⁹ meters. For example, the visible spectrum (Figure 6), ranges from the deep red with a wavelength of 700 nm to violet with a wavelength of 400 nm.

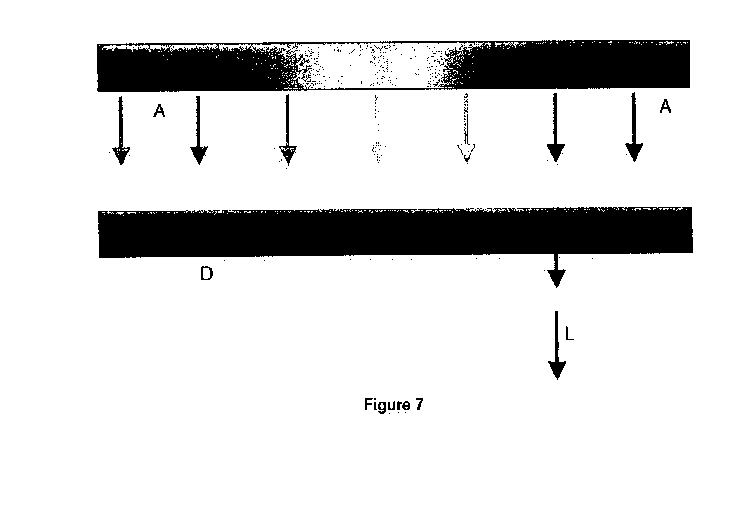

There are many types of light filters which allow different colors of the light spectrum to go through, based on their different wavelengths. There are green, blue, red, antethermic, etc. Most commercially available filters are not very discriminating and are unable to choose only one wavelength. Specially designed and very costly filters would be required to achieve a high level of discrimination. The colposcopy of this invention requires a special narrow band pass interference filter as illustrated in Figure 7-D. That filter is known as the "exciter" filter and it is designed to pass light with a wavelength of 488 nanometers (Figure 7-L).

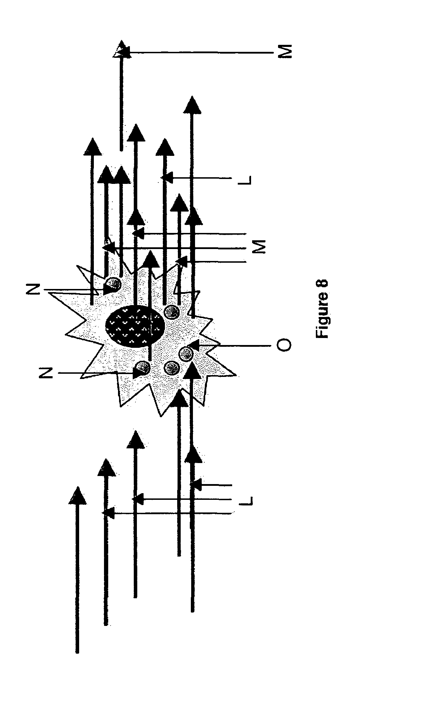

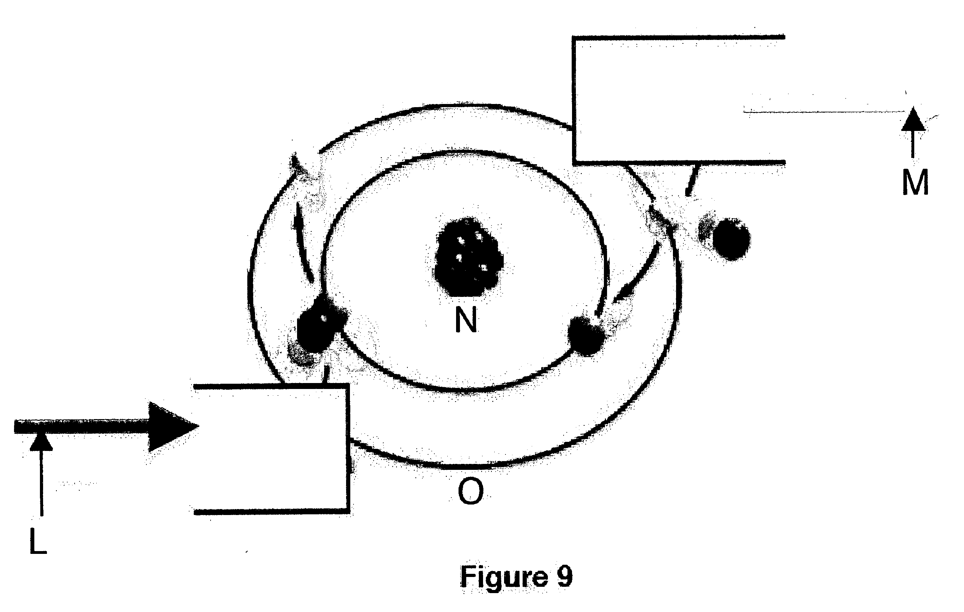

Below, we describe the excitation of the fluorochrome in the colposcopy of this invention. First, the fluorochrome is applied in the patient's cervix. Tissue cells infected with HPV absorb the fluorochrome while non-infected tissue cells do not absorb it (Figure 8). A light beam of 488 nm wavelength (Figure 8-L) hits the fluorochrome inside the infected cells (Figures 8-N and 8-O), and infected tissue. The flourochrome then absorbs in the infected tissues the light energy generated and emits light in different wavelengths as compared with the light with which it was irradiated (Figure 8-M). The physical phenomenon of fluorescence is produced: The tissue cells fluoresce. The energy of the light irradiated by the excitement filter at 488nm (Figure 7-D), is absorbed by atoms of fluorescein isothiocyanate (Figure 9) inside the infected cells (Figures 8-N, 9-N), exciting its electrons, changing its spin (Figure 9), and causing the emission of photons. A photon is produced when an electron's spin changes. In this case, photons with a wavelength of 520 nm (Figure 9-M) are produced. The photon's wavelength is significantly different from the wavelength with which the infected cells and tissue were irradiated originally. That effect is known as the Stokes-Adams Principle.

The following figure shows where, within the apparatus of this invention, is located the exciting filter, which is an easy to handle system and which can be set and ready to use (Figures 1-D and 2-D). The light beam goes through the exciting filter, and the ray goes through a first prism where it is sent to the frontal lens (Figures 1-B2, 2-B2, 4-B2) of the object, which condenses the light, to then illuminate the point of the tissue being observed within the patient's lower genital tract (Figures 1-F, 4-F).

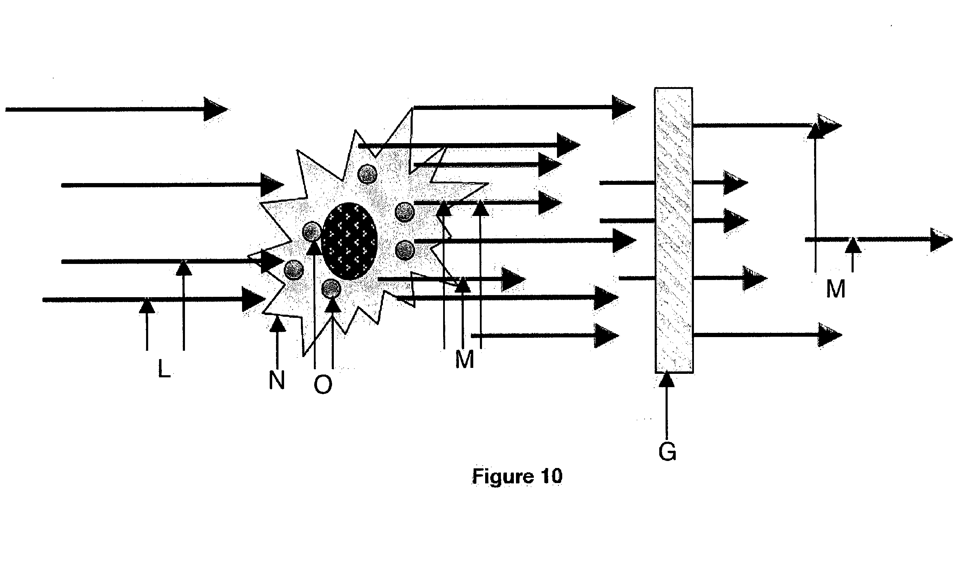

Figures 8 and 10 provide a specific example of epithelial cells organelles when they are excited. The luminescent energy irradiates the fluorochrome. That energy is then absorbed and converted in a light beam of a different wavelength, as described before. The frontal lens then collects the energy and transmits it to the suppressing filters (Figures 1-G, 2-G, 10-G), located inside the observational optic system of the Colposcope. That system comprises a set of two objective lenses (Figure 1-I) which send the image to the dull prisms (Figure 1-J). The prisms then invert the image so that it can be analyzed through oculars (Figures 1-K, and 2-K). The colposcope of this invention also comprises a suppressor filter which sends the light beam to a video camera to be observed by the user through a monitor.

Suppressor filters are used to locate and see the fluorochrome in the infected cells and tissues. Specifically, a band pass filter allowing light with a wavelength of 520 nanometers to pass through is used (Figures 1-G, 2-G and 10-G). Two filters are required in the tri-dimensional stereoscopic system with oculars set up at 7 degrees of opening for each eye, and one filter is required for the video-photographic equipment. The reason for needing the filters is to allow passage of light of 520 nm wavelength to be seen or recorded. That way the infected cell and tissue becomes unusually bright and can be distinguished from the healthy cell and tissue.

Having shown the epithelial cells being excited, the suppressor filter only allows for the light to go through at 520 nm. This way the zones where the fluorochrome is not captured will be dark, and, by contrast, the user will be able to clearly distinguish and see the zones where the flourochrome was captured, which coincide with the zones affected by the HPV-caused lesions also known as Dysplasia. Within the aforementioned context, a positive test is obtained when the HPV infected areas capture the fluorochrome, and a negative test result is obtained when there is no flourochrome.

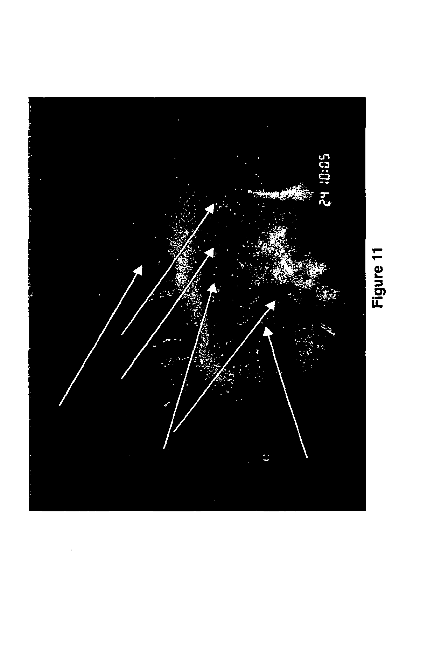

Figure 11 illustrates a view of HPV induced lesions using the apparatus of the present invention. It is important to note that certain mucus tissue also shows us as fluorescent due to the cellular discarded materials, which should not be confused with the lesions indicated by the arrows. Figure 11 involved a 50 year old patient which presented stage 3, positive biopsies. In that particular case, ordinary colposcopy failed to detect the HPV lesions.

### Preferred Embodiment of Use of the Invention

In its preferred embodiment, the method of using the present invention uses the fluorochrome *Fluorescein Isothiocyanate* for several important reasons. That system is commonly used by ophthalmologists to conduct retinal fluorangiography, and in a solution to see corneal lesions. Ophthalmologists perform those procedures using an actinic lamp which has a cobalt filter and a wide spectral emission. The actinic lamp used by ophthalmologists does not have suppressor filters. The method of using this invention uses a fluorochrome because, among other reasons, it does not present toxic or adverse effects to humans. In the clinical context, the method comprises the steps of:
- setting the patient in the gynecological position;
- inserting the plastic vaginal speculum to avoid undesirable reflections;
- visually locating the patient's cervix;
- taking a vaginal pH swab (frequently HPV infections present with other bacterial illnesses or parasitical infections which are characterized by alkaline pH):
- taking samples of the endocervix with the citobrush;
- taking samples of the ectocervix with the Ayre Spatula (speculum) or device;
- uniformly preparing the sample trays to be sent to the laboratory to confirm the diagnosis of the method of this invention, if the ordering physician so requires;
- applying a 5% aqueous solution of acetic acid into the patient's cervix for one minute. The acidic solution has two functions: (1) bleaching the intraepithelial cervical neoplasia's cells; and (2) bonds the fluorochrome to the cells;
- conducting a clear field colposcopy, which has approximately 70% accuracy and requires a rather long training time for prospective users;
- introducing the green filter to see the vascular changes in the ordinary Colposcopy;
- making the video-photographic logs to be able to compare images later;
- uniformly applying the fluorescein isothiocyanate to the patient's cervix for one minute;
- activating the colposcope's exciting filter.
- activating the colposcope's suppressor filter, resulting in a "new" image which is obtained instantly;
- making video-photographic logs of the "new" image using the camera mounted on the bolster; and
- in case of a positive result, and according to the degree and severity of the lesions disclosed by the "new" image, sending an affected area tissue sample in an Eppendorf tube with the citobrush to a molecular biology laboratory for viral typing.

The method disclosed herein in the clinical context takes between 15 and 20 minutes.

## Claims

1. A colposcopy apparatus adapted to detect specific lesions produced by the human papilloma virus in tissue in the female lower genital tract, the apparatus comprising a colposcope utilizing a source of actinic light (A), a first narrow band pass filter (D) placed in use between the source and the tissue, and a second narrow band pass filter (G) for light emitted from the tissue in use, **characterised in that** the second narrow band pass filter (G) is designed to allow the passage of light having a wavelength of 520 nanometers, and **in that** the second narrow band pass filter consists of three separate narrow band pass filters (G), two of the narrow band pass filters being arranged in a tridimensional stereoscopic system with a 7 degree opening for each eye and the third of the narrow band pass filters being provided for a video-photographic system.

2. The colposcopy apparatus of claim 1 in which the source of actinic light (A) is aligned with a collector lens (B), the position of the source (A) being adjustable relative to the collector lens along two axes whereby the most intensified luminosity may be obtained.

3. The colposcopy apparatus of claim 1 in which the first narrow band pass filter (D) is designed to allow the passage of light having a wavelength of 488 nanometers.

## Patentansprüche

1. Kolposkopievorrichtung, angepasst zur Erkennung spezifischer Läsionen, die durch das humane Papillomavirus in Gewebe des weiblichen unteren Genitaltrakts hervorgerufen werden, die Vorrichtung umfassend ein Kolposkop, das mit einer aktinischen Lichtquelle (A) arbeitet, einen ersten Schmalbandfilter (D), der bei der Verwendung zwischen der Quelle und dem Gewebe platziert wird, und einen zweiten Schmalbandfilter (G) für Licht, das bei der Verwendung vom Gewebe emittiert wird, **dadurch gekennzeichnet, dass** der zweite Schmalbandfilter (G) so ausgelegt ist, dass er Licht mit einer Wellenlänge von 520 Nanometern passieren lässt, und dadurch, dass der zweite Schmalbandfilter aus drei separaten Schmalbandfiltern (G) besteht, wobei zwei der Schmalbandfilter in einem dreidimensionalen stereoskopischen System mit einer 7-Grad-Öffnung für jedes Auge angeordnet sind und der dritte der Schmalbandfilter für ein videofotografisches System bereitgestellt wird.

2. Kolposkopievorrichtung gemäß Anspruch 1, bei der die aktinische Lichtquelle (A) an einer Kollektorlinse (B) ausgerichtet ist, wobei die Position der Lichtquelle (A) entlang von zwei Achsen im Verhältnis zu der Kollektorlinse eingestellt werden kann, wodurch die intensivste Helligkeit erzielt werden kann.

3. Kolposkopievorrichtung gemäß Anspruch 1, bei der der erste Schmalbandfilter (D) so ausgelegt ist, dass er Licht mit einer Wellenlänge von 488 Nanometern passieren lässt.

## Revendications

1. Un appareil de colposcopie adapté pour détecter des liaisons spécifiques produites par le virus du papillome humain dans le tissu du tractus génital inférieur féminin, l'appareil comportant un colposcope utilisant une source de lumière actinique (A), un premier filtre passe-bande étroit (D) placé lors de l'utilisation entre la source et le tissu, et un deuxième filtre passe-bande étroit (G) pour la lumière émise en provenance du tissu lors de l'utilisation, **caractérisé en ce que** le deuxième filtre passe-bande étroit (G) est conçu pour permettre le passage d'une lumière ayant une longueur d'onde de 520 nanomètres, et **en ce que** le deuxième filtre passe-bande étroit est constitué de trois filtres passe-bande étroits distincts (G), deux des filtres passe-bande étroits étant agencés dans un système stéréoscopique tridimensionnel avec une ouverture à 7 degrés pour chaque oeil et le troisième des filtres passe-bande étroits étant prévu pour un système vidéo-photographique.

2. L'appareil de colposcopie de la revendication 1 dans lequel la source de lumière actinique (A) est alignée avec une lentille collectrice (B), la position de la source (A) étant réglable relativement à la lentille collectrice sur deux axes grâce à quoi la luminosité la plus intensifiée peut être obtenue.

3. L'appareil de colposcopie de la revendication 1 dans lequel le premier filtre passe-bande étroit est conçu pour permettre le passage d'une lumière ayant une longueur d'onde de 488 nanomètres.
